Europäisches Patentamt

European Patent Office

Office européen des brevets

(19)

(11) Veröffentlichungsnummer : **0 324 794 B1**

# (12) EUROPÄISCHE PATENTSCHRIFT

(45) Veröffentlichungstag der Patentschrift :
**29.01.92 Patentblatt 92/05**

(21) Anmeldenummer : **87907302.1**

(22) Anmeldetag : **06.11.87**

(86) Internationale Anmeldenummer :
**PCT/DE87/00507**

(87) Internationale Veröffentlichungsnummer :
**WO 88/03803 02.06.88 Gazette 88/12**

(51) Int. Cl.⁵ : **A61K 31/465,** A61K 9/48,
A61K 9/66

(54) **ORAL EINZUNEHMENDE KAPSEL MIT EINEM NIKOTINHALTIGEN, FLÜSSIGEN MITTEL.**

Die Akte enthält technische Angaben, die nach dem Eingang der Anmeldung eingereicht wurden und die nicht in dieser Patentschrift enthalten sind.

(30) Priorität : **18.11.86 DE 3639418**

(43) Veröffentlichungstag der Anmeldung :
**26.07.89 Patentblatt 89/30**

(45) Bekanntmachung des Hinweises auf die Patenterteilung :
**29.01.92 Patentblatt 92/05**

(84) Benannte Vertragsstaaten :
**AT BE CH DE FR GB IT LI LU NL SE**

(56) Entgegenhaltungen :
**BE-A- 899 037**
**DE-A- 3 241 437**
**LU-A- 65 929**

(73) Patentinhaber :
**FORSCHUNGSGESELLSCHAFT RAUCHEN**
**UND GESUNDHEIT MBH**
**Frauenthal 2**
**W-2000 Hamburg 13 (DE)**

(72) Erfinder : **ADLKOFER, Franz**
**Parallelstrasse 18**
**W-1000 Berlin 45 (DE)**

(74) Vertreter : **Jander, Dieter, Dipl.-Ing. et al**
**Dipl.-Ing. Dieter Jander Dr.-Ing. Manfred**
**Böning Patentanwälte Leistikowstrasse 2**
**W-1000 Berlin 19 (DE)**

EP 0 324 794 B1

EP 0 324 794 B1

## Beschreibung

Die Erfindung bezieht sich auf einer oral einzunehmende Zerbeißk apsel mit einem nikotinhaltigen, flüssigen Mittel.

Eine oral zu nehmende Kapsel ist bereits bekanntgeworden (DE-OS 36 06 892). Bei dieser Kapsel soll der Inhalt langsam und kontinuierlich im Munde der einzunehmenden Person durch ein winziges Loch in der Kapselwand aufgrund von osmotischem Druck herausgedrückt werden (Seite 13, Abs. 1), was jedoch nicht funktioniert.

In der Schrift ist nichts über die Nikotinmenge in der Kapsel, nichts über etwaige Zusatzstoffe und auch nichts über den pH-Wert des Mittels offenbart.

Der Erfindung liegt die Aufgabe zugrunde, eine Kapsel der eingangs erwähnten Art zu schaffen, die es der die Kapsel einnehmenden Person ermöglicht, Nikotin bezüglich Menge und Geschwindigkeit ähnlich wie beim Zigarettenrauchen aufzunehmen.

Diese Aufgabe wird erfindungsgemäß gelöst durch eine oral einzunehmende Zerbeißkapsel mit einem flüssigen Mitteln enthaltend Nikotin in einer Menge zwischen 0,1 - 10 mg, vorzugsweise 2-5 mg, sowie Zusatzstoffe zur Geschmaks- und Verträglichkeitsverbesserung bei einem pH-Wert des Mittels zwischen 7 und 10. Bevorzugt hat der Kapselinhalt ein Volumen von 0,1 - 2,5 ml, insbesondere von 0,1- 1,0 ml.

Wird eine solche Kapsel eingenommen und zerbissen, ergießt sich deren Inhalt über die Mundschleimhäute mit der Folge, daß das Nikotin über diese in den körper und in den Kreislauf gelangt. Die Person erfährt einen kräftigen Nikotinstoß, wobei das Nikotin rasch das Gehirn erreicht. Durch den vorgegebenen Nikotingehalt der kapsel und den vorgegebenen pH-Wert des Mittels ist die aufgenommene Nikotinmenge bestimmt. Die Menge ist mit der Menge vergleichbar, die ein Raucher aufnimmt, wenn er eine Zigarette raucht und den Rauch inhaliert.

Die vorgesehenen Zusatzstoffe sorgen dafür, daß der unangenehme und reizende Geschmack des Nikotins gemildert wird.

Wichtig ist, daß die nikotinhaltige Flüssigkeit einige Minuten im Mund verbleibt. Innerhalb von 5 Minuten wird bei diesem Vorgehen aus 1,5 ml Lösung, die 3,0 mg Nikotin enthält und einen pH-Wert von 8,0 aufweist, eine Nikotinmenge von bis zu 1,5 mg resorbiert. Diese Menge entspricht ungefähr der, die beim Rauchen einer mittelstarken Zigarette aufgenommen wird. Die in Lösung verbleibende Nikotinmenge kann nach einer gewissen Zeit, die von der die kapsel einnehmenden Person bestimmt wird, ausgespuckt oder verschluckt werden. Durch Schlucken kommt es zu einem zusätzlichen Nikotinanstieg in Blut und Gewebe, welcher im Gegensatz zum Arstieg nach Nikotinresorption im Mund langsam erfolgt und somit die Erstwirkung verstärkt.

Aus der DE-OS 32 41 437 ist ein nikotinhaltiges Spray zur oralen Anwendung bekannt. Die Nikotinapplikation mittels eines Sprays ist jedoch relativ unangenehm.

Aus der GB-PS 15 28 391 ist bekannt, daß die Dosiseinheit von Nikotin, auf die Mundschleimhäute einwirkend, zwischen 0,1 und 10,0 mg liegt. Jedoch handelt es sich hierbei um ein Aerosol, das in den Mund eingesprüht wird. Ferner sind keine Zusatzstoffe zur Geschmacks- und Verträglichkeitsverbesserung vorgesehen, und schließlich ist auch nichts über den pH-Wert des Mittels offenbart.

Aus der DE-OS 34 01 763 ist ein in die Nase zu verabreichendes nikotinhaltiges Mittel bekannt. Auch diese Applikation ist unangenehm, da die Nasenschleimhaut empfindlicher als die Mundschleimhaut ist. Außerdem ist das Verbleiben des Mittels im Nasenraum nicht gewährleistet.

Aus der DE-OS 34 38 284 ist ein nikotinabgebendes Depotpflaster bekannt. Hiermit ist jedoch ein Nikotinstoß nicht möglich. Die Nikotinaufnahme erfolgt sehr langsam und unkontrolliert.

Aus "Transdermal Administration of Nicotine" ("Drug and Alcohol Dependence", 13, 1984, Seiten 209-213) ist ebenfalls ein über die Haut einwirkendes Nikotinmittel bekannt. Auch hierbei ist die stoßweise Aufnahme von Nikotin nicht möglich.

Aus der BE-PS 899 037 ist ein Lutschbonbon bekannt, das Nikotin enthält. Auch mit diesem ist eine stoßweise Verabreichung des Nikotins nicht möglich.

Bekannt sind auch Kapseln mit irgendwelchen Arzneimitteln (F. Gstirner, "Einführung in die Verfahrenstechnik der Arzneiformung", 1973, Seiten 177-181). Jedoch handelt es sich hierbei nicht um nikotinhaltige Kapseln.

Weiterhin sind auch Zerbeißkapseln bekannt (B. Helwig, "Moderne Arzneimittel", 1980, Seite 815). Aber auch hierbei handelt es sich nicht um nikotinhaltige Kapseln.

Zahlreiche wissenschaftliche Befunde sprechen dafür, daß Nikotin in der Dosis, in der es beim Rauchen aufgenommen wird, keine krankmachenden Eigenschaften besitzt. Insbesondere scheint es nicht bei der Entstehung der sogenannten Raucherkrankungen mitzuwirken.

Nikotin übt vielfältige Wirkungen auf das Nervensystem und die psychovegetativen Funktionen aus. Es kann zur Raucherentwöhnung und bei folgenden Indikationen als Medikament eingesetzt werden:

2

Übergewicht
Morbus Parkinson
Morbus Alzheimer
Angstneurosen
Zerebralsklerose
Colitis ulcerosa.

All diese Indikationen sind über die Acetycholin-Rezeptoren miteinander verknüpft.

Die pharmakologische Bedeutung des Nikotins bei den verschiedenen hier genannten Indikationen beruht auf seinen Eigenschaften als Cholinergikum mit vor allem zentralen, aber auch peripheren Wirkungen. Über seine Bindung an cholinergen Rezeptoren übt Nikotin auch einen indirekten Einfluß auf die Freisetzung der meistbekannten Neurotransmitter aus, da das cholinerge System mit den anderen Transmittersystemen in einer Wechselbeziehung steht. Alle hier erwähnten Krankheiten werden durch eines oder mehrere der betroffenen Rezeptorsysteme beeinflußt.

## Patentansprüche

1. Oral einzunehmende Zerbeißkapsel mit einem flüssigen Mittel, enthaltend Nikotin in einer Menge zwischen 0,1 -10 mg sowie Zusatzstoffe zur Geschmacks- und Verträglichkeitsverbesserung bei einem pH-Wert des Mittels zwischen 7 und 10.

2. Zerbeißkapsel nach Anspruch 1, enthaltend Nikotin in einer Menge zwischen 2 und 5 mg.

3. Zerbeißkapsel nach Anspruch 1, dadurch **gekennzeichnet**, daß der Kapselinhalt ein Volumen von 0,1 - 2,5 ml, insbesondere von 0,1 - 1,0 ml hat.

4. Verwendung der Zerbeißkapsel nach einem der Ansprüche 1 bis 3 zur Herstellung eines Arzneimittels zur Raucherentwöhnung und gegen Übergewicht, Morbus Parkinson, Morbus Alzheimer, Angstneurosen, Zerebralsklerose und Colitis ulcera.

## Claims

1. An orally administered bitable capsule with a fluid medium containing nicotine in an amount between 0.1 and 10 mg. and with additives to improve the taste and tolerability, the pH-value of the medium between 7 and 10.

2. A bitable capsule according to Claim 1 which contains nicotine in an amount of between 2 and 5 mg.

3. A bitable capsule according to Claim 1, characterized in that the capsule contents have a volume of 0.1 - 2.5 ml, preferably 0,1 - 1.0 ml.

4. A bitable capsule according to any one of Claims 1-3 when used for the preparation of a medicinal product to stop smoking and to treat obesity, Parkinson's disease, Alzheimer's disease, anxiety neuroses, cerebral sclerosis and ulcerative colitis.

## Revendications

1. Capsule à administration orale, à briser avec les dents, comportant un milieu liquide contenant de la nicotine en une quantité comprise entre 0,1 et 10 mg, de préférence 2 et 5 mg, ainsi que des additifs d'amélioration du goût et de l'acceptabilité, pour une valeur du pH du milieu comprise entre 7 et 10.

2. Capsule à briser avec les dents suivant la revendication 1, contenant la nicotine en une quantité comprise entre 2 et 5 mg.

3. Capsule à briser avec les dents suivant la revendication 1, caractérisée en ce que le contenu de la capsule a un volume compris entre 0,1 et 2,5 ml, notamment entre 0,1 et 1,0 ml.

4. Utilisation de la capsule à briser avec les dents suivant l'une des revendications 1 à 3 pour la préparation d'un médicament servant à la désaccoutumance du fumeur et contre l'éxcès de poids, la maladie de Parkinson, la maladie de Alzheimer, les phobies névrotiques, la sclérose cérébrale et la colite ulcéreuse.